# EUROPEAN PATENT SPECIFICATION

(11) **EP 3 142 542 B1**
(45) Date of publication and mention of the grant of the patent: **06.01.2021**
(21) Application number: 15721959.3
(22) Date of filing: 28.04.2015
(51) Int. Cl.: A61B 5/00, A61B 8/08, A61B 5/0205, A61B 5/08, G16H 50/20, G16H 50/30

(54) **AUTOMATIC DIFFERENTIAL DIAGNOSIS OF WORSENING HEART FAILURE**
AUTOMATISCHE DIFFERENTIALDIAGNOSE DER VERSCHLECHTERUNG VON HERZVERSAGEN
DIAGNOSTIC DIFFÉRENTIEL AUTOMATIQUE D'UNE AGGRAVATION D'UNE DÉFAILLANCE CARDIAQUE

(30) Priority: 15.05.2014 US 201461993824 P
(43) Date of publication of application: 22.03.2017
(73) Proprietor: Cardiac Pacemakers, Inc., St. Paul, MN 55112-5798 (US)
(72) Inventor: ZHANG, Yi, Plymouth, Minnesota 55446 (US); WARIAR, Ramesh, Blaine, Minnesota 55449 (US); THAKUR, Pramodsingh Hirasingh, Woodbury, Minnesota 55129 (US); AVERINA, Viktoria A., Shoreview, Minnesota 55126-2334 (US); AN, Qi, Blaine, Minnesota 55449 (US); SWEENEY, Robert J., Woodbury, Minnesota 55125 (US); THOMPSON, Julie A., Circle Pines, Minnesota 55014 (US)
(74) Representative: Pfenning, Meinig & Partner mbB
(86) International application number: PCT/US2015/027947
(87) International publication number: WO 2015/175207

(56) References cited:
- WO-A1-2012/059477
- WO-A2-2006/125097
- US-A1- 2008 157 980
- US-A1- 2011 301 982
- US-A1- 2011 306 845
- KENTON L. ANDERSON ET AL: "Diagnosing heart failure among acutely dyspneic patients with cardiac, inferior vena cava, and lung ultrasonography", THE AMERICAN JOURNAL OF EMERGENCY MEDICINE, vol. 31, no. 8, 1 August 2013 (2013-08-01) , pages 1208-1214, XP055197615, ISSN: 0735-6757, DOI: 10.1016/j.ajem.2013.05.007
- HELEN C. HANCOCK ET AL: "High prevalence of undetected heart failure in long-term care residents: findings from the Heart Failure in Care Homes (HFinCH) study", EUROPEAN JOURNAL OF HEART FAILURE, vol. 15, no. 2, 1 February 2013 (2013-02-01), pages 158-165, XP055197613, ISSN: 1388-9842, DOI: 10.1093/eurjhf/hfs165

## Description

### TECHNICAL FIELD

This document relates generally to medical devices, and more particularly, to systems, devices and methods for detecting and monitoring heart failure decompensation.

### BACKGROUND

Congestive heart failure (CHF) is a major health problem and affects over five million people in the United States alone. CHF is the loss of pumping power of the heart, resulting in the inability to deliver enough blood to meet the demands of peripheral tissues. CHF patients typically have enlarged heart with weakened cardiac muscles, resulting in reduced contractility and poor cardiac output of blood.

CHF is usually a chronic condition, but can occur suddenly. It can affect the left heart, right heart or both sides of the heart. If CHF affects the left ventricle, signals that control the left ventricular contraction are delayed, and the left and right ventricles do not contract simultaneously. Non-simultaneous contractions of the left and right ventricles further decrease the pumping efficiency of the heart.

### OVERVIEW

Frequent monitoring of CHF patients and timely detection of events indicative of heart failure (HF) decompensation status can help prevent worsening HF in CHF patients, hence reducing cost associated with HF hospitalization. Additionally, identification of patient at an elevated risk of developing future HF events such as worsening HF can help ensure timely treatment, thereby improving the prognosis and patient outcome. Identifying and safely managing the patients having risk of future HF events can avoid unnecessary medical intervention and reduce healthcare cost.

Ambulatory medical devices can be used for monitoring HF patient and detecting HF decompensation events. Examples of such ambulatory medical devices can include implantable medical devices (IMD), subcutaneous medical devices, wearable medical devices or other external medical devices. The ambulatory or implantable medical devices can include physiologic sensors which can be configured to sense electrical activity and mechanical function of the heart, or physical or physiological variables associated with the signs and symptoms of worsening HF. The medical device can optionally deliver therapy such as electrical stimulation pulses to a target area, such as to restore or improve the cardiac function or neural function.

Identification of patient at an elevated risk of developing future HF events such as worsening of HF can help ensure timely treatment, thereby improving the prognosis and patient outcome. Identifying and safely managing the patients at low risk of future HF events can avoid unnecessary medical intervention and reduce healthcare cost. Ideally, an HF detection method can include one or more of a high sensitivity, a high specificity, a high positive predictive value (PPV), or a high negative predictive value (NPV). In the context of risk stratification for HF decompensation event, the sensitivity can represent the accuracy of identifying high-risk patients (e.g., those more likely to develop a future HF decompensation episode). The specificity can represent the accuracy of identifying low-risk patients (e.g., those less likely to develop a future HF decompensation episode). However, factors such as difference of medical conditions across patients and/or disease progression within a patient can negatively affect the performance of a HF event detector or a HF stratifier. For example, some detected HF events are false positive detections: rather than being caused by worsening HF such as a HF decompensation, these events are resulted from other diseases or medical conditions such as pulmonary disease, cardiac arrhythmias, infections, or surgeries. The false-positive detections may also be caused by confounding factors such as malfunction of a part of an implantable device system. The false positive detections can lead to a low specificity or a low PPV of detecting worsening HF events. On the other hand, the false positive diseases or conditions can be comorbid conditions associated with or precipitate worsening HF events, yet in worsening HF detection a further differential diagnosis of these false-positive diseases or conditions often lacks or remains unclear. This may result in inappropriate therapy and patient management when a false positive detection occurs.

Therefore, the present inventors have recognized that there remains a considerable need of systems and methods that can detect target physiologic events indicative of worsening HF or identify CHF patients with elevated risk of developing future events of worsening HF with improved accuracy and reliability. In particular, a differential diagnosis between worsening of HF and other diseases or medical conditions, including identifying causes other than worsening HF that result in the pathophysiologic manifestation indicating deteriorated patient status, can be clinically beneficial in facilitating prompt clinical decision and intervention.

The invention is as specified in claim 1.

Various embodiments described herein can help improve detection of worsening HF by recognizing or diagnosing other diseases or medical conditions that may yield similar pathophysiological manifestations. For example, a system provide a differential diagnosis between worsening HF and other candidate conditions. The system, such as an ambulatory medical device (AMD), can comprise a patient information receiver circuit that receives patient information including one or more physiologic signals, and a physiologic data analyzer circuit configured to detect a respective physiologic feature from the received physiologic signals. The system can include a differential diagnosis circuit that generates one or more signal metrics, receive two or more candidate conditions associated with the change in patient physical or physiological status, and determine a respective diagnostic score for the two or more candidate conditions. The diagnostic score can indicate likelihood the change in the patient physical or physiologic status being caused by the corresponding candidate condition. The system can include a user interface that produces a presentation of the detected physiologic features and the diagnostic scores associated with the candidate conditions.

A method can include receiving patient information including one or more physiologic signals obtained from a patient, and detecting physiologic feature from the physiologic signals. The method includes processes of generating one or more signal metrics indicative or correlative of a change in the patient physical or physiologic status, receiving two or more candidate conditions associated with the change in patient physical or physiological status, and determining a respective diagnostic score for each candidate condition. The diagnostic score can indicate likelihood the change in the patient physical or physiologic status being caused by the corresponding candidate condition. The method includes generating a presentation including the detected physiologic features and the diagnostic scores associated with the two or more candidate conditions.

This Overview is an overview of some of the teachings of the present application and not intended to be an exclusive or exhaustive treatment of the present subject matter. Further details about the present subject matter are found in the detailed description and appended claims. Other aspects of the present disclosure will be apparent to persons skilled in the art upon reading and understanding the following detailed description and viewing the drawings that form a part thereof, each of which are not to be taken in a limiting sense. The scope of the present disclosure is defined by the appended claims and their legal equivalents.

Document WO 2006/125097 relates to data mining and more particularity, to systems and methods for mining and/or using clinical information from patient medical records.

### BRIEF DESCRIPTION OF THE DRAWINGS

Various embodiments are illustrated by way of example in the figures of the accompanying drawings. Such embodiments are demonstrative and not intended to be exhaustive or exclusive embodiments of the present subject matter.
FIG. 1 illustrates an example of a cardiac rhythm management (CRM) system and portions of the environment in which the CRM system operates.
FIG. 2 illustrates an example of an automatic HF differential diagnosis circuit.
FIG 3 illustrates an example of a signal metric generator circuit.
FIG. 4 illustrates an example of a diagnostic score generator circuit.
FIG. 5 illustrates an example of a method for differential diagnosis between worsening HF and other diseases of medical conditions.
FIG. 6 illustrates an example of a rule-based model for differential diagnosis between worsening HF and other disease or medical conditions.

### DETAILED DESCRIPTION

Disclosed herein are systems, devices, and methods for automatically generating differential diagnosis decisions of worsening of HF such as HF decompensation. The differential diagnosis decision can include diagnostic scores each associated with respective candidate condition such as worsening HF event, pulmonary disease, an atrial or ventricular arrhythmias, an indication of a surgical procedure, or an infection. The diagnostic score associated with a particular candidate condition can be generated using physiologic signals or other physiologic information obtained from a patient. The diagnostic score indicates likelihood of a pathophysiological manifestation, such as a deteriorated patient status, being caused by the candidate condition. The automatically generated differential diagnosis can be presented to an end-user in guiding the patient treatment and management decisions.

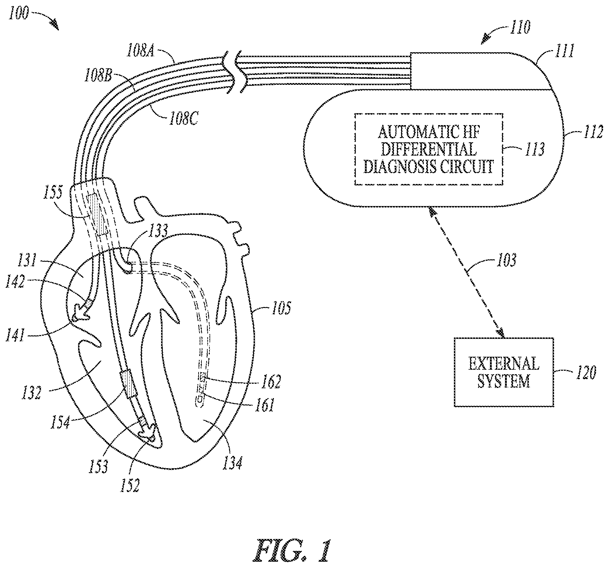

FIG. 1 illustrates an example of a Cardiac Rhythm Management (CRM) system 100 and portions of an environment in which the CRM system 100 can operate. The CRM system 100 can include an ambulatory medical device, such as an implantable medical device (IMD) 110 that can be electrically coupled to a heart 105 such as through one or more leads 108A-C, and an external system 120 that can communicate with the IMD 110 such as via a communication link 103. The IMD 110 may include an implantable cardiac device such as a pacemaker, an implantable cardioverter-defibrillator (ICD), or a cardiac resynchronization therapy defibrillator (CRT-D). The IMD 110 can include one or more monitoring or therapeutic devices such as a subcutaneously implanted device, a wearable external device, a neural stimulator, a drug delivery device, a biological therapy device, a diagnostic device, or one or more other ambulatory medical devices. The IMD 110 may be coupled to, or may be substituted by a monitoring medical device such as a bedside or other external monitor.

As illustrated in FIG. 1, the IMD 110 can include a hermetically sealed can 112 that can house an electronic circuit that can sense a physiological signal in the heart 105 and can deliver one or more therapeutic electrical pulses to a target region, such as in the heart, such as through one or more leads 108A-C. The CRM system 100 can include only one lead such as 108B, or can include two leads such as 108A and 108B.

The lead 108A can include a proximal end that can be configured to be connected to IMD 110 and a distal end that can be configured to be placed at a target location such as in the right atrium (RA) 131 of the heart 105. The lead 108A can have a first pacing-sensing electrode 141 that can be located at or near its distal end, and a second pacing-sensing electrode 142 that can be located at or near the electrode 141. The electrodes 141 and 142 can be electrically connected to the IMD 110 such as via separate conductors in the lead 108A, such as to allow for sensing of the right atrial activity and optional delivery of atrial pacing pulses. The lead 108B can be a defibrillation lead that can include a proximal end that can be connected to IMD 110 and a distal end that can be placed at a target location such as in the right ventricle (RV) 132 of heart 105. The lead 108B can have a first pacing-sensing electrode 152 that can be located at distal end, a second pacing-sensing electrode 153 that can be located near the electrode 152, a first defibrillation coil electrode 154 that can be located near the electrode 153, and a second defibrillation coil electrode 155 that can be located at a distance from the distal end such as for superior vena cava (SVC) placement. The electrodes 152 through 155 can be electrically connected to the IMD 110 such as via separate conductors in the lead 108B. The electrodes 152 and 153 can allow for sensing of a ventricular electrogram and can optionally allow delivery of one or more ventricular pacing pulses, and electrodes 154 and 155 can allow for delivery of one or more ventricular cardioversion/defibrillation pulses. In an example, the lead 108B can include only three electrodes 152, 154 and 155. The electrodes 152 and 154 can be used for sensing or delivery of one or more ventricular pacing pulses, and the electrodes 154 and 155 can be used for delivery of one or more ventricular cardioversion or defibrillation pulses. The lead 108C can include a proximal end that can be connected to the IMD 110 and a distal end that can be configured to be placed at a target location such as in a left ventricle (LV) 134 of the heart 105. The lead 108C may be implanted through the coronary sinus 133 and may be placed in a coronary vein over the LV such as to allow for delivery of one or more pacing pulses to the LV. The lead 108C can include an electrode 161 that can be located at a distal end of the lead 108C and another electrode 162 that can be located near the electrode 161. The electrodes 161 and 162 can be electrically connected to the IMD 110 such as via separate conductors in the lead 108C such as to allow for sensing of the LV electrogram and optionally allow delivery of one or more resynchronization pacing pulses from the LV.

The IMD 110 can include an electronic circuit that can sense a physiological signal. The physiological signal can include an electrogram or a signal representing mechanical function of the heart 105. The hermetically sealed can 112 may function as an electrode such as for sensing or pulse delivery. For example, an electrode from one or more of the leads 108A-C may be used together with the can 112 such as for unipolar sensing of an electrogram or for delivering one or more pacing pulses. A defibrillation electrode from the lead 108B may be used together with the can 112 such as for delivering one or more cardioversion/defibrillation pulses. In an example, the IMD 110 can sense impedance such as between electrodes located on one or more of the leads 108A-C or the can 112. The IMD 110 can be configured to inject current between a pair of electrodes, sense the resultant voltage between the same or different pair of electrodes, and determine impedance using Ohm's Law. The impedance can be sensed in a bipolar configuration in which the same pair of electrodes can be used for injecting current and sensing voltage, a tripolar configuration in which the pair of electrodes for current injection and the pair of electrodes for voltage sensing can share a common electrode, or tetrapolar configuration in which the electrodes used for current injection can be distinct from the electrodes used for voltage sensing. In an example, the IMD 110 can be configured to inject current between an electrode on the RV lead 108B and the can housing 112, and to sense the resultant voltage between the same electrodes or between a different electrode on the RV lead 108B and the can housing 112. A physiologic signal can be sensed from one or more physiological sensors that can be integrated within the IMD 110. The IMD 110 can also be configured to sense a physiological signal from one or more external physiologic sensors or one or more external electrodes that can be coupled to the IMD 110. Examples of the physiological signal can include one or more of electrocardiogram, intracardiac electrogram, arrhythmia, heart rate, heart rate variability, intrathoracic impedance, intracardiac impedance, arterial pressure, pulmonary artery pressure, left atrial pressure, RV pressure, LV coronary pressure, coronary blood temperature, blood oxygen saturation, one or more heart sounds, physical activity or exertion level, physiologic response to activity, posture, respiration, body weight, or body temperature.

The arrangement and functions of these leads and electrodes are described above by way of example and not by way of limitation. Depending on the need of the patient and the capability of the implantable device, other arrangements and uses of these leads and electrodes are possible.

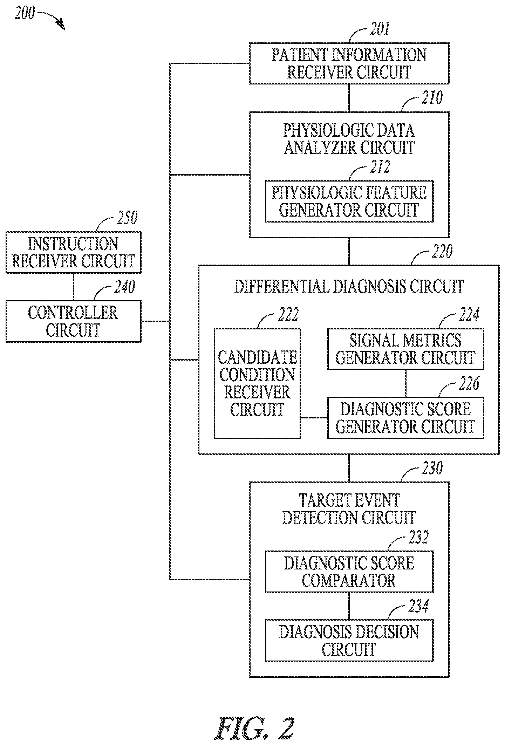

As illustrated, the CRM system 100 can include an automatic heart failure (HF) differential diagnosis circuit 113. The automatic HF differential diagnosis circuit 113 can receive one or more patient information sources such as one or more physiologic signals, and generate one or more signal metrics from the received physiologic information. The automatic heart failure (HF) differential diagnosis circuit 113 can be coupled to one or more ambulatory physiologic sensors deployed on or within the patient and communicated with the IMD 110, such as electrodes on one or more of the leads 108A-C and the can 112, or ambulatory physiologic sensors deployed on or within the patient and communicated with the IMD 110. The automatic HF differential diagnosis circuit 113 can receive two or more candidate conditions, and determine for each candidate condition a respective diagnostic score using the one or more signal metrics. The diagnostic score can indicate likelihood of the change in the patient physical or physiologic status being caused by the corresponding candidate condition. Examples of the automatic HF differential diagnosis circuit 113 are described below, such as with reference to FIGS. 2-4.

The external system 120 can allow for programming of the IMD 110 and can receive information about one or more signals acquired by IMD 110, such as can be received via a communication link 103. The external system 120 can include a local external IMD programmer. The external system 120 can include a remote patient management system that can monitor patient status or adjust one or more therapies such as from a remote location.

The communication link 103 can include one or more of an inductive telemetry link, a radio-frequency telemetry link, or a telecommunication link, such as an internet connection. The communication link 103 can provide for data transmission between the IMD 110 and the external system 120. The transmitted data can include, for example, real-time physiological data acquired by the IMD 110, physiological data acquired by and stored in the IMD 110, therapy history data or data indicating IMD operational status stored in the IMD 110, one or more programming instructions to the IMD 110 such as to configure the IMD 110 to perform one or more actions that can include physiological data acquisition such as using programmably specifiable sensing electrodes and configuration, device self-diagnostic test, or delivery of one or more therapies.

The automatic HF differential diagnosis circuit 113 may be implemented at the external system 120, which can be configured to perform HF risk stratification or HF event detection, such as using data extracted from the IMD 110 or data stored in a memory within the external system 120. Portions of automatic HF differential diagnosis circuit 113 may be distributed between the IMD 110 and the external system 120. The differential diagnosis circuit 113 needs not be limited to a hardware circuit, but can include a microprocessor that is configured by being provided instructions that, when performed, carry out the functions of the circuit.

Portions of the IMD 110 or the external system 120 can be implemented using hardware, software, or any combination of hardware and software. Portions of the IMD 110 or the external system 120 may be implemented using an application-specific circuit that can be constructed or configured to perform one or more particular functions, or can be implemented using a general-purpose circuit that can be programmed or otherwise configured to perform one or more particular functions. Such a general-purpose circuit can include a microprocessor or a portion thereof a microcontroller or a portion thereof or a programmable logic circuit, or a portion thereof For example, a "comparator" can include, among other things, an electronic circuit comparator that can be constructed to perform the specific function of a comparison between two signals or the comparator can be implemented as a portion of a general-purpose circuit that can be driven by a code instructing a portion of the general-purpose circuit to perform a comparison between the two signals. While described with reference to the IMD 110, the CRM system 100 could include a subcutaneous medical device (e.g., subcutaneous ICD, subcutaneous diagnostic device), wearable medical devices (e.g., patch based sensing device), or other external medical devices.

FIG. 2 illustrates an example of an automatic HF differential diagnosis circuit 200, which can be an embodiment of the automatic HF differential diagnosis circuit 113. The automatic HF differential diagnosis circuit 200 can be implemented as a hardware circuit, or it can include a microprocessor configured by being provided instructions that, when performed, carry out the functions of the circuit. The automatic HF differential diagnosis circuit 200 can also be implemented in an external system such as a patient monitor configured for providing the patient's diagnostic information to an end-user. The automatic HF differential diagnosis circuit 200 can include one or more of a patient information receiver circuit 201, a physiologic data analyzer circuit 210, a differential diagnosis circuit 220, a controller circuit 240, and an instruction receiver circuit 250. The automatic HF differential diagnosis circuit 200 can optionally include a target event detection circuit 230.

The patient information receiver circuit 201 can be configured to receive patient information including one or more physiologic signals obtained from a patient. The physiologic signals can be sensed using one or more ambulatory physiologic sensors, or using one or more external sensors or testing devices communicatively coupled to the patient information receiver circuit 201. Examples of such a physiologic signal can include one or more of surface or subcutaneous electrocardiograph (ECG), electrograms such as sensed using electrodes from one or more of the leads 108A-C or the can 112, heart rate, heart rate variability, arrhythmia information, intrathoracic impedance, intracardiac impedance, arterial pressure, pulmonary artery pressure, left atrial pressure, RV pressure, LV coronary pressure, coronary blood temperature, body core temperature, blood oxygen saturation, one or more heart sounds, systolic time intervals, heart sound based cardiac time intervals, impedance based cardiac time intervals, physiologic response to activity, physical activity, night-time restlessness, patient's posture, patient's weight, apnea hypopnea index, one or more respiration signals such as a respiration rate signal, a tidal volume signal, a minute ventilation signal, or rapid shallow breathing index (RR/TV) signal. The physiologic signals can also include one or more of brain natriuretic peptide (BNP), blood panel, sodium and potassium levels, glucose level and other biomarkers and bio-chemical markers. The physiologic signals can also include device therapy statistics such as a percentage of biventricular or left-ventricular only pacing in patient with ambulatory medical devices.

The patient information received by the information receive circuit 201 can additionally receive diagnostic information collected by an ambulatory device. Examples of the diagnostic information can include event counters, pacing mode switches, lead impedance or other device or lead integrity test data, among others. The patient information receiver circuit 201 can also receive information about patient present and past health data and medical records including, for example, past and present medication, surgical procedures, or other medical history information. The patient information receiver circuit 201 can receive other types of information such as patient demographic information, or social and behavioral information (e.g., smokers or non-smokers).

In addition to or as an alternative of acquiring the patient information using physiologic sensors, the patient information receiver circuit 201 can be coupled to the storage device, such as an electronic medical record (EMR) system, and retrieve from the storage device one or more patient historical physiologic signals in response to a command signal. The command signal can be issued by an end-user such as via an input device coupled to the instruction receiver 250, or generated automatically by the system in response to a specified event.

The physiologic data analyzer circuit 210 can include one or more sub-circuits that can perform signal conditioning or pre-processing, including signal amplification, digitization, or filtering, on the one or more physiologic signals. The physiologic data analyzer circuit 210 can include a physiologic feature generator circuit 212 configured to detect, from each of the one or more pre-processed physiologic signals, a respective physiologic feature indicative of patient physical or physiologic status. Examples of physiologic features can include mean, median, or other central tendency measures; a histogram of the signal intensity; a plurality of signal trends over time; one or more signal morphological descriptors; one or more signal change or rate of change features; one or more signal change or rate of change features, or signal power spectral density at a specified frequency range. The physiologic features can include components corresponding to physiologic activities. For example, the electrocardiogram or electrogram features can include P wave, R wave, T wave, QRS complex, or other components representing depolarization, hyperpolarization, repolarization, or other electrophysiological properties of the myocardium. The heart sound features can include relative timing (such as with respect to R wave), amplitude, or morphologic characteristics of one or more of S1, S2, S3, or S4 heart sounds. The impedance features can include maximum, minimum, mean, variance, rate of change, or other statistical or morphological features. The respiration signal features can include respiration rate, respiration depth, tidal volume, minute ventilation, rapid shallow breathing index (RR/TV), or other descriptors.

The differential diagnosis circuit 220 can automatically generate diagnostic information including likelihood of the patient physical or physiologic status indicating or being caused by a worsening of a present disease or medical condition. The differential diagnosis circuit 220 can include a candidate condition receiver circuit 222, a signal metrics generator circuit 224, and a diagnostic score generator circuit 226.

The candidate condition receiver circuit 222 can receive two or more candidate conditions such as via an input device coupled to the instruction receiver 250, or from a storage device such as a memory circuit that stores two or more pre-determined candidate conditions. The candidate conditions can include diseases or medical conditions that are likely to cause or correlate to the patient physical or physiological manifestations. As an example, the candidate conditions can include worsening HF such as HF decompensation. Other examples of candidate conditions can include pulmonary diseases (such as chronic obstructive pulmonary disease, pneumonia, or bronchitis), sleep disordered breathing, atrial or ventricular arrhythmias (such as atrial fibrillation, atrial tachycardia, ventricular tachycardia, or ventricular fibrillation), a renal disease, hypertension, diabetes, or other comorbidities of HF or diseases or conditions triggering or precipitating worsening HF. The candidate conditions can also include a clinical events such as a surgical procedure (such as cardiac or valvular surgery, ablation, implant of a ventricular assisted device, implantable medical device replacement, device pocket or implantable lead revision), or an infection (such as infection associated with an implantable medical device and lead system), among others.

The signal metrics generator circuit 224 can be coupled to the physiologic feature generator circuit 212, and generate one or more signal metrics using the physiologic features. The signal metrics can indicate a change in the patient physical or physiologic status. The signal metrics generator circuit 224 can perform multiple measurements of the physiologic features such as during a specified period of time or when certain condition is met, and compute one or more signal metrics using the multiple measurements of the physiologic features. The signal metrics can include a statistical metric or a morphological metric derived from the multiple measurements of the physiologic features.

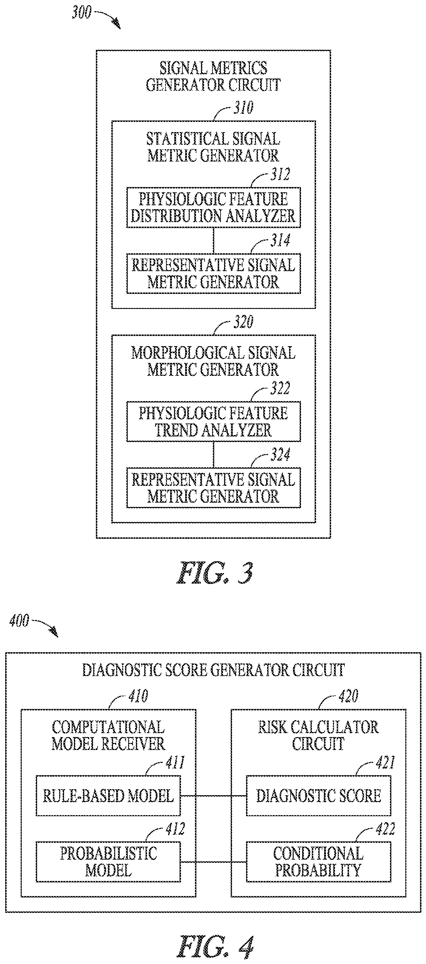

In one example, the signal metrics generator circuit 224 can receive a plurality of measurements of thoracic impedance value computed using a thoracic impedance signal such as sensed by the patient information receiver circuit 201. The cyclic variation of the thoracic impedance can be indicative of patient respiration. A statistical metric, such as a central tendency measure of the plurality of impedance measurements, can be computed to provide a statistical measure of patient prespiration strength, respiration rate, or respiration pattern. In another example, the signal metrics generator circuit 224 can receive a plurality of measurements of S3 heart sound intensity determined by using heart sound signals such as sensed by the patient information receiver circuit 201. A morphologic metric such as a change in S3 heart sound intensity from a baseline value can be computed. Such S3 intensity metric can be indicative of cardiac diastolic function change which is predictive of worsening HF. In various examples, sustained elevated values for a signal metric, or variability of the signal metric, may also be predictive of worsening HF. Examples of the signal metrics generator circuit 224 are discussed below, such as with reference to FIG. 3.

The diagnostic score generator circuit 226 can be coupled to the candidate condition receiver circuit 222 and the signal metrics generator circuit 224. The diagnostic score generator circuit 226 can determine a respective diagnostic score for the two or more candidate conditions received by the candidate condition receiver circuit 222. The diagnostic score can indicate likelihood of the change in the patient physical or physiologic status being caused by the corresponding candidate condition. The diagnostic score can be computed using one or more signal metrics such as provided by the signal metrics generator circuit 224 and a computational model. Examples of the diagnostic score generator circuit 226 are discussed below, such as with reference to FIG. 4.

The optional target event detection circuit 230 can detect a target event as one of the candidate conditions. A target event can include a physiologic event indicative of an onset of a disease, or a change (such as worsening or improvement) of a disease state. Examples of target event or condition can include an event indicative of HF decompensation status, change in HF status such as worsening HF, pulmonary edema, or myocardial infarction. The optional target event detection circuit 230 can include a diagnostic score comparator circuit 232 that can compare the diagnostic scores associated with different candidate conditions. A most probable diagnosis decision can be made by the diagnosis decision circuit 234, such as by selecting from the two or more candidate conditions one candidate condition that has the highest diagnostic score.

The controller circuit 240 can control the operations of the patient information receiver circuit 201, the physiologic data analyzer circuit 210, the differential diagnosis circuit 220, a controller circuit 240, an instruction receiver circuit 250, and optionally the target event detector circuit 230, as well as the data flow and instructions between these components. The controller circuit 240 can receive external programming input from the instruction receiver circuit 250 to control one or more of receiving patient information, sensing of physiologic signals and extracting physiologic features, receiving candidate conditions, generating signal metrics, computing diagnostic scores, or optionally forming a diagnosis decision. The instruction receiver circuit 250 can include a user interface configured to present programming options to the user and receive user's programming input. The user interface can provide a presentation of the detected physiologic features and the diagnostic scores associated with the two or more candidate conditions. Optionally, the user interface can provide the presentation including the most probable diagnosis. In an example, at least a portion of the instruction receiver circuit 250, such as the user interface, can be implemented in the external system 120.

FIG. 3 illustrates an example of a signal metric generator circuit 300, which can be an embodiment of the signal metrics generator circuit 224. The signal metric generator circuit 300 can include one or both of a statistical signal metric generator 310 and a morphological signal metric generator 320.

The statistical signal metric generator 310 can be configured to generate a statistical parameter from the plurality of physiologic feature measurements such as provided by the physiologic data analyzer circuit 210. Examples of the statistical parameter can include mean, median, or other central tendency measures, standard deviation, variance, correlation, covariance, or other higher-order statistics computed from the plurality of physiologic feature measurements, among others.

The statistical parameter of the physiologic feature measurements can also be determined using a statistical distribution of the plurality of physiologic feature measurements. In an example as illustrated in FIG. 3, the statistical signal metric generator 310 can include a physiologic feature distribution analyzer 312 and a representative signal metric generator 314. The physiologic feature distribution analyzer 312 can generate a histogram of the plurality of physiologic feature measurements. In another example, the physiologic feature distribution analyzer 312 can further approximate the histogram of the physiologic feature measurement by a statistical distribution function. The histogram or the approximated statistical distribution function each indicates the frequency of occurrence of a physiologic feature value during which the physiologic feature measurements are taken.

The representative signal metric generator 314 can determine a representative signal metric using the plurality of physiologic feature measurements and a threshold value associated with the statistical distribution or the histogram of the physiologic feature measurements. An example of such a threshold value is a percentile rank (PR) that indicates relative number of physiologic feature measurements (e.g., percentage of the plurality of the physiologic feature measurements) with values falling below or equal to the representative metric. The representative signal metric generator 314 can receive a specified PR from an end-user such as via the instruction receiver circuit 250, or alternatively from a data storage unit such as a memory circuit where a pre-determined PR is stored.

The morphological signal metric generator 320 can include a physiologic feature trend analyzer 322 and a representative signal metric generator 324. The feature trend analyzer 322 can be configured to create a physiologic feature trend using a plurality of physiologic feature measurements. A physiologic feature trend can be a time-series signal representing temporal variation of the physiologic feature. The representative signal metric generator 324 can generate one or more morphological features from a physiologic feature trend signal. Examples of the morphological parameters can include maximum or minimum within a specified period, amount of change within a specified period, positive or negative slope that indicates the rate of increase or rate of decrease in the physiologic feature, signal power spectral density at a specified frequency range, among other morphological descriptors. In an example, the signal metric can be computed as a difference between a first statistical measure of the physiologic feature computed from a first time window and a second statistical measure of the physiologic feature computed from a second time window. The first and the second statistical measures can each include a mean, a median, a mode, a percentile, a quartile, or other measures of central tendency of the signal metric values in the respective time window. In an example, the second time window can be longer than the first window, and at least a portion of the second time window precedes the first time window in time. The second statistical measure can represent a baseline value of the signal metric. In some examples, the signal metrics can include a composite signal metric computed using two or more physiological signals.

FIG. 4 illustrates an example of a diagnostic score generator circuit 400, which can be an embodiment of the diagnostic score generator circuit 226. The diagnostic score generator circuit 400 can include a computational model receiver 410 and a risk calculator circuit 420.

The computational model receiver circuit 410 can include one or more computational models used for computing a diagnostic score. A computational model can be a specified set of processor-executable instructions stored in a memory. Examples of the computational models can include a rule-based model, a decision tree model, a regression model, a neural network model, a random forest, a voting model, a fuzzy logic model, or a support vector machine model, among other machine learning models. A computational model can be configured with specified components and structure. For example, a decision tree model can include structural components of nodes, paths, and tree levels.

The risk calculator circuit 420 can apply the one or more signal metrics to a computation model and calculate a corresponding diagnostic score. As illustrated in FIG. 4, the computational model receiver can include one or both of a rule-based model 411 and a probabilistic model 412. A rule-based model can comprise a plurality of rules each defining a criterion for one or more of signal metrics, and the risk calculator circuit 420 can calculate a diagnostic score 421 in response to the one or more signal metrics respectively meeting a specified criterion.

In one example, the rule-based model can include rules for differential diagnosis between worsening of HF and pulmonary diseases. The model can utilize signal metrics including a change in intrathoracic total impedance value (ITTI) from a reference value (ΔITTI = ITTI - ITTI_{Ref}), a change in respiration rate (RR) from a reference respiration rate (ΔRR = RR - RR_{Ref}), a rate of change of RR (ΔRR/Δt), and a change in a heart sound (HS) component such as S3 heart sound intensity from a reference level (Δ||S3|| = ||S3|| - ||S3||_{Ref}). The ITTI can include a direct-current (DC) component of a wide-band intrathoracic impedance signal such as measured using two or more electrodes from one or more of the leads 108A-C or the can 112. In an example, voltage across electrode 153 and can 112 can be measured in response to electric current injected across electrode 154 and can 112, and the ITTI can be computed using Ohm's law. The reference levels, including ITTI_{Ref}, RR_{Ref}, and ||S3||_{Ref}, can be determined using measurements of respective sensor signals during baseline when the patient is deemed free of the candidate conditions. Alternatively, the reference levels can be dynamically determined as a moving-average of respective signal metrics over a moving time window.

The model comprises rules of assigning a higher diagnostic score to the candidate condition of "worsening HF" if (1) ITTI substantially decreases from the reference level by at least a threshold value, which indicates substantial intrathoracic fluid accumulation; (2) RR substantially increases from RR_{Ref} by at least a threshold value, and ΔRR/Δt is within a threshold range, which indicates a gradual onset of increase in respiration rate; and (3) ||S3|| substantially increases from the reference level by at least a threshold value. The model comprises rules of assigning a higher diagnostic score to the candidate condition of "pulmonary disease" if (1) RR substantially increases from the RR_{Ref} by at least a threshold value and ΔRR/Δt exceeds a threshold range, which indicates a sudden onset in rise of respiration rate; or (2) ||S3|| is within a threshold range around ||S3||_{Ref}. As an example, the threshold for ΔRR can be approximately an increase of 2-4 breaths per minute, the threshold for ΔITTI can be approximately a decrease of 8-10% from a reference level, and the threshold for Δ||S3|| can be approximately an increase of 0.3-0.5 milli-g. Based on the diagnostic score 421, the diagnostic decision circuit 234 can present the diagnostic decision of "worsening HF" or "pulmonary disease" to the end-user as the most probable diagnosis.

In another example, the rule-based model can include rules for differential diagnosis between worsening of HF and atrial fibrillation (AF). The model can utilize signal metrics including a change or a rate of change in a HS component, such as Δ||S3|| or a change or rate of change in S1 heart sound (Δ||S1|| and Δ||S1||/Δt, respectively), a rate of change in heart rate (HR) from a reference level (ΔHR/Δt = (HR-HR_{Ref})/ Δt), and a change in rapid shallow breathing index (RSBI) from a reference level (ΔRSBI = RSBI - RSBI_{Ref}). The RSBI, defined as the ratio of respiratory frequency (number of breaths per minutes) to tidal volume, indicates the number of breaths per minute per liter. The model comprises the rules of assigning a higher diagnostic score to the candidate condition of "AF" than to "worsening HF" if (1) ΔHR/Δt is positive and exceeds a threshold, indicating sudden increase in HR; (2) Δ||S3|| is positive and exceeds a threshold, indicating ||S3|| substantially increases from the reference level; (3) Δ||S1||/Δt is negative and exceeds a threshold, indicating a sudden decrease in S1 intensity; and (4) ΔRSBI is positive and exceeds a threshold, indicating a substantial increase in RSBI from the reference level likely due to intensified rapid and shallow breathing. As an example, the threshold for ΔHR/Δt can be approximately an increase of at least 10 bpm within a range of 1-3 days, the threshold for Δ||S3|| can be approximately an increase of 0.3-0.5 milli-g, and the threshold for ΔRSBI can be approximately an increase of 10-15% from a reference RSBI level. AF may also be monitored by AF burden, which is defined as amount of time spent on AF during a specified time window, such as 24 hours. Based on the diagnostic score 421, the diagnostic decision circuit 234 can present the diagnostic decision of "AF" to the end-user as the most probable diagnosis.

In another example, the rule-based model can include rules for differential diagnosis between worsening of HF and surgical procedures such as revision of a part of implantable lead system or pocket for an implantable medical device, implant of ventricular assist device, or other cardiac or valvular surgery. The model can utilize signal metrics including a rate of change in ITTI from a reference value such as determined as an moving-average over a time window, that is, ΔITTI/Δt = (ITTI - ITTI_{Ref})/ Δt, and a rate of change in tidal volume (TV) from a reference level, that is, ΔTV/Δt = (TV -TV_{Ref})/Δt. The model comprises the rules of assigning a higher diagnostic score to the candidate condition of "surgical procedures" than to "worsening HF" if (1) ΔITTI/Δt is negative and exceeds a threshold value, indicating a sudden decrease in intrathoracic impedance; and (2) ΔTV/Δt is negative and exceeds a threshold, indicating a sudden decrease in depth of respiration. As an example, the threshold for ΔITTI/Δt can be approximately a decrease of 15-20% from a reference level within a range of 1-3 days, and the threshold for ΔTV/Δt can be approximately a decrease of 15-20% from a reference level within a range of 1-3 days. Based on the diagnostic score 421, the diagnostic decision circuit 234 can present the diagnostic decision of "surgical procedure" to the end-user as the most probable diagnosis.

In yet another example, the rule-based model can include rules for differential diagnosis between worsening of HF and infections associated with a portion of the implantable medical device or an implantable lead. The model can utilize signal metrics including a change in a heart sound component, such as Δ||S3||, from a reference level, a change in ITTI value from a reference level (ΔITTI), and rate of change in HR, RR, or TV (ΔHR/Δt, ΔRR/Δt, or ΔTV/Δt, respectively). A sudden change in vital signs such as HR, RR, or TV can indicate worsening general health condition which can be caused by infection; and an additional detected change in ITTI can indicate the infection being confined within tissues at or near device pocket or lead system. The model comprises the rules of assigning a higher diagnostic score to the candidate condition of "device/lead infection" if (1) ΔHR/Δt or ΔRR/Δt is positive and exceeds a threshold, or ΔTV/Δt is negative and exceeds a threshold, indicating respectively a sudden increase in HR or in RR, or a sudden decrease in TV; (2) ITTI is within a threshold range around ITTI_{Ref}, indicating non-significant intrathoracic fluid accumulation; and (3) ||S3|| is within a threshold range around ||S3||_{Ref}. As an example, the threshold for ΔHR/Δt can be approximately an increase of at least 5 bpm within a range of 1-3 days, the threshold for ΔRR/Δt can be approximately an increase of at least 4 breaths/minute within a range of 1-3 days, the threshold for ΔTV/Δt can be approximately a decrease of 15-20% from a reference level within a range of 1-3 days, the threshold for ΔITTI can be approximately 15-20% a reference level, and the threshold for Δ||S3|| can be approximately 0.3-0.5 milli-g around the reference level. Based on the diagnostic score 421, the diagnostic decision circuit 234 can present the diagnostic decision of "infection" to the end-user as the most probable diagnosis.

The rule-based model can also detect events other than a particularized disease or a medical condition. In an example, the rule-based model can include rules for detecting patient's being out of a specified range from a location such as where an external patient data receiver or communicator is positioned. The detection of patient being out of range may indicate, for example, the patient being hospitalized, if the data receiver or the communicator fails to properly receive data packets according to a specified data communication criterion. In another example, the rule-based model can include rules for detecting changes to one or more programming parameters of the implantable device. The diagnostic decision circuit 234 can present the corresponding decisions of "patient out of range" or "device reprogrammed" to the end-user as the most probable cause associated with the patient condition.

As an alternative or in addition to the plurality of rules each setting a respective criterion for one particular signal metric, the rule-based model can include a plurality of rules each defining a criterion for a comparison between two different signal metrics. The comparison can include relative signal metric intensity, relative amount of change or rate of change between two signal metrics with respect to their respective reference or baseline level, or relative timing of the change in signal metrics. In an example, the rule-based model can utilize signal metrics including a first timing of a change in apnea-hypopnea index (T_AHI), a second timing of a change in intrathoracic total impedance value (T_ΔITTI), and a third timing of a change in a HS component such as S3 heart sound intensity (T_Δ||S3||). The model comprises the rules of assigning a higher diagnostic score to the candidate condition of "sleep disordered breathing (SDB)" if T_AHI precedes T_ΔITTI and T_Δ||S3||, which indicates disordered breathing patterns during sleep develop before any significant change in sensors responses such as thoracic impedance or HS intensity. Based on the diagnostic score 421, the diagnostic decision circuit 234 can present the diagnostic decision of "SDB" to the end-user as the most probable diagnosis.

In another example, the rule-based model can further comprise a signal metric being a timing of a detected ventricular arrhythmia episode such as a ventricular tachycardia or ventricular fibrillation (T_VT/VF). The ventricular arrhythmia episode can be detected by an ambulatory medical device. The model comprises the rules of assigning a higher diagnostic score to the candidate condition of "VT/VF" if T_VT/VF precedes T_ΔITTI and T_Δ||S3||, which indicates a VT/VF episode is developed before any significant change in sensors responses such as thoracic impedance or HS intensity. Based on the diagnostic score 421, the diagnostic decision circuit 234 can present the diagnostic decision of "VTNF" to the end-user as the most probable diagnosis.

The computational model receive can also receive a probabilistic model 412. The probabilistic model can include, for each of the one or more signal metric, a descriptor of statistical distribution of the one or more signal metrics. Examples of the probabilistic model can include a Markov model, a hidden Markov model, a Bayesian network model, or a stochastic grammar model, among other stochastic graphical models. In an example, the probabilistic model 412 can be a Bayesian network model that encodes dependencies and causal relationships among the signal metrics and the candidate condition using probability measurements. The Bayesian networks can be constructed using prior knowledge including statistical distribution of signal metrics and statistical distributions of candidate conditions which can be estimated using data from a patient population. The diagnostic score computed by the risk calculator circuit 420 can include a probability measure of the patient experiencing worsening of one of the candidate conditions or developing in the future a new candidate condition. In an example, the probability measure can be a conditional probability 422, which indicates the probability of the patient experiencing worsening of one of the candidate conditions or developing a future candidate condition given that the patient has manifested physical or pathophysiological presentations as indicated by the one or more signal metrics.

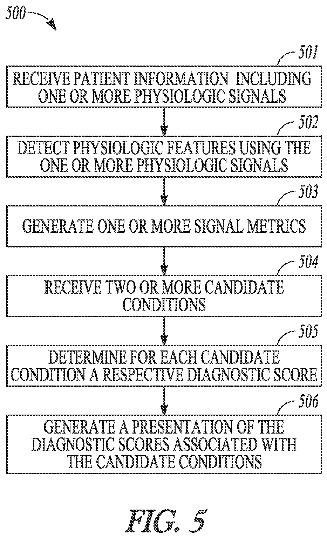

FIG 5 illustrates an example of a method 500 for differential diagnosis between worsening HF and other diseases of medical conditions. The method 500 can be implemented and operate in an ambulatory medical device or in a remote patient management system. In an example, the method 500 can be performed by automatic HF differential diagnosis circuit 113 implemented in the IMD 110, or the external device 120 which can be in communication with the IMD 110.

The method 500 can include a process of receiving patient information at 501. The patient information can include one or more physiologic signals obtained from a patient, such as sensed using one or more ambulatory physiologic sensors, external sensors, or testing devices. Examples of such a physiologic signal can include one or more of surface or subcutaneous electrocardiograph (ECG), electrograms, heart rate, heart rate variability, arrhythmia information, intrathoracic impedance, intracardiac impedance, arterial pressure, pulmonary artery pressure, left atrial pressure, RV pressure, LV coronary pressure, coronary blood temperature, blood oxygen saturation, one or more heart sounds, systolic time intervals, heart sound based cardiac time intervals, impedance based cardiac time intervals, physiologic response to activity, apnea hypopnea index, one or more respiration signals such as a respiration rate signal, a tidal volume signal, a minute ventilation signal, or rapid shallow breathing index (RR/TV) signal. The physiologic signals can also include one or more of brain natriuretic peptide (BNP), blood panel, sodium and potassium levels, glucose level and other biomarkers and bio-chemical markers.

The patient information received at 510 can also include diagnostic information collected by an ambulatory device, such as event counters, pacing mode switches, or lead impedance or other device or lead integrity test data, among others. Additionally or alternatively, the patient information received at 510 can include patient health information such as past and present medication and therapy information, or medical history information. In some examples, the patient information can be stored in a storage device such as an electronic medical record (EMR) system, and retrievable from the storage device in response to a command signal.

At 502, one or more physiologic features can be detected from each of the one or more physiologic signals. The physiologic features can include mean, median, or other central tendency measures; a histogram of the signal intensity; a plurality of signal trends over time; one or more signal morphological descriptors; one or more signal change or rate of change features; one or more signal change or rate of change features, or signal power spectral density at a specified frequency range. The physiologic features can include components corresponding to physiologic activities. For example, the electrocardiogram or electrogram features can include P wave, R wave, T wave, QRS complex, or other components representing depolarization, hyperpolarization, repolarization, or other electrophysiological properties of the myocardium. The heart sound (HS) signal features can include relative timing (such as with respect to R wave), amplitude, or morphologic characteristics of one or more of S1, S2, S3, or S4 heart sounds. The thoracic impedance features can include maximum, minimum, mean, variance, rate of change, or other statistical or morphological features. The respiration signal features can include respiration rate, respiration depth, tidal volume, minute ventilation, rapid shallow breathing index (RSBI), apnea-hypopnea index (AHI), or other signal features.

The physiologic features can be used to generate one or more signal metrics at 503. The signal metrics can indicate a change in the patient physical or physiologic status. The signal metrics can include statistical or a morphological metrics derived from multiple measurements of the physiologic features, such as measured during a specified period of time or when certain condition is met.

At 504, two or more candidate conditions can be received. The candidate conditions can include diseases or medical conditions that are likely to cause, or correlate to, the patient physical or pathophysiologic manifestations. Examples of candidate conditions can include worsening HF, pulmonary diseases (such as chronic obstructive pulmonary disease, pneumonia, or bronchitis), sleep disordered breathing, atrial or ventricular arrhythmias (such as atrial fibrillation, atrial tachycardia, ventricular tachycardia, or ventricular fibrillation), a renal disease, hypertension, diabetes, or other comorbidities of HF or diseases or conditions triggering or precipitating worsening HF. The candidate conditions can also include a clinical events such as a surgical procedure (such as cardiac or valvular surgery, ablation, implant of a ventricular assisted device, implantable medical device replacement, device pocket or implantable lead revision), or an infection (such as infection associated with an implantable medical device and lead system), among others.

At 505, a diagnostic score can be determined for the two or more candidate conditions. The diagnostic score can indicate likelihood of the change in the patient physical or physiologic status being caused by the corresponding candidate condition. The diagnostic score can be computed using one or more signal metrics and a computational model. A computational model can be a specified set of processor-executable instructions stored in a memory. Examples of the computational models can include a rule-based model, a decision tree model, a regression model, a neural network model, a random forest, a voting model, a fuzzy logic model, or a support vector machine model, among other machine learning models. The computational models can also include a probabilistic model that determines a diagnostic score for a signal metric using a statistical distribution of the one or more signal metrics. Examples of the probabilistic model can include a Markov model, a hidden Markov model, a Bayesian network model, or a stochastic grammar model, among other stochastic graphical models.

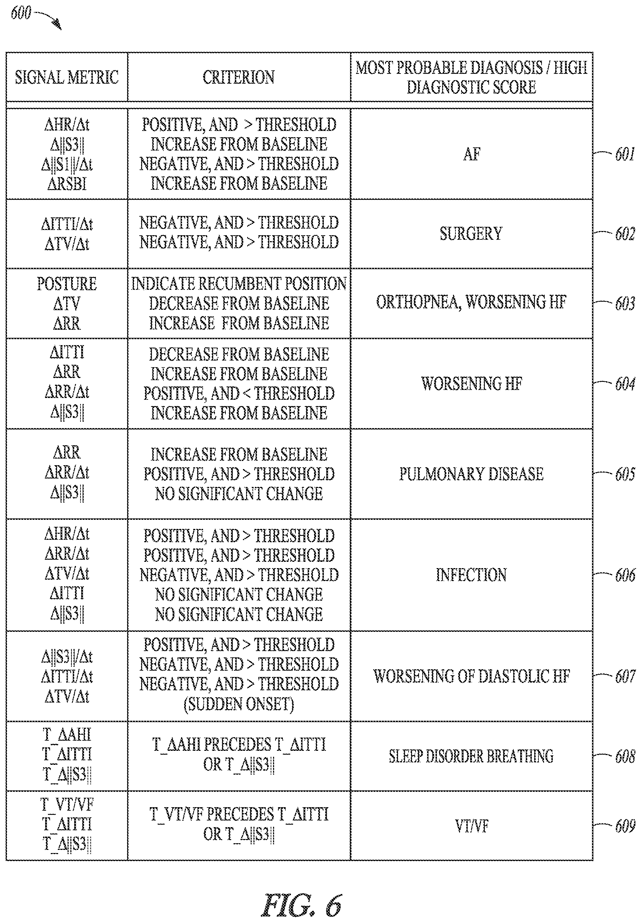

A rule-based model can comprise a plurality of rules each defining a criterion for one or more of signal metrics, and a diagnostic score can be determined in response to the one or more signal metrics respectively meeting a specified criterion. The rule-based model can include a plurality of rules each defining a criterion for a comparison between two different signal metrics, such as relative timing of the change in signal metrics between the two signal metrics. Examples of differential diagnosis between worsening of HF and other disease using a rule-based model are discussed below, such as with reference to FIG. 6.

At 506, a presentation of the diagnostic scores associated with the candidate conditions can be generated. Optionally, a most probable diagnosis can be generated and presented to an end-user. In an example, the most probable diagnosis can be selected from the two or more candidate conditions as the one that is associated with the highest diagnostic score.

FIG. 6 illustrates an example of a rule-based model 600 for differential diagnosis between worsening HF and other disease or medical conditions. The rule-based model 600 can be constructed as a lookup table or an association map that establishes a mapping from a signal metric meeting a specified criterion to a most probable diagnosis or a diagnostic score assigned to a particular candidate condition. The rule-based model 600 can be an embodiment of determining respective diagnostic score for an individual candidate condition, such as with reference to process 505 in FIG. 5. The rule-based model 600 can be performed by automatic HF differential diagnosis circuit 113 implemented in the IMD 110.

As illustrated in FIG. 6, the rule-based model 600 can include a rule 601 of diagnosing AF or assigning a high diagnostic score to AF. The rule 601 employs signal metrics including a change or a rate of change in a HS component, such as Δ||S3|| or a change or rate of change in S1 heart sound (Δ||S1|| and Δ||S1||/Δt, respectively), a rate of change in heart rate from a reference level (AHR/At = (HR-HR_{Ref})/ Δt), and a change in rapid shallow breathing index (RSBI) from a reference level (ΔRSBI = RSBI - RSBI_{Ref}). The RSBI can be defined as the ratio of respiratory frequency (number of breaths per minutes) to tidal volume. According to the rule 601, a higher diagnostic score can be assigned to the candidate condition of "AF" if (1) ΔHR/Δt is positive and exceeds a threshold, indicating sudden increase in HR; (2) Δ||S3|| is positive and exceeds a threshold, indicating ||S3|| substantially increases from the reference level; (3) Δ||S1||/Δt is negative and exceeds a threshold, indicating a sudden decrease in S1 intensity; and (4) ΔHR/Δt is positive and exceeds a threshold, indicating a sudden increase in heart rate.

Rule 602 can be used for differentiating diseases like worsening of HF from a surgical procedure which may lead to similar pathophysiological manifestations in a patient. Examples of the surgical procedures can include revision of a part of implantable lead system or pocket for an implantable medical device, implant of ventricular assist device, or other cardiac or valvular surgery. The rule 602 can employ signal metrics including a rate of change in intrathoracic total impedance value (ITTI) from a reference value such as determined as an moving-average over a time window, that is, AITTI/At = (ITTI - ITTI_{Ref})/ Δt, and a rate of change in tidal volume (TV) from a reference level, that is, ΔTV/Δt = (TV -TV_{Ref})/Δt. The model comprises the rules of assigning a higher diagnostic score to the candidate condition of "surgical procedures" than to "worsening HF" if (1) ΔITTI/Δt is negative and exceeds a threshold value, indicating a sudden decrease in intrathoracic impedance; and (2) ΔTV/Δt is negative and exceeds a threshold, indicating a sudden decrease in tidal volume.

Rule 603 can be used for detecting orthopnea, and assigning a high diagnostic score to worsening HF on the basis of a manifestation of orthopnea being highly indicative of worsening of HF. The rule 603 uses signal metrics including a posture, and other signal metrics indicative of elevated respiration effort, such as an increase in RR or a decrease in TV. A higher diagnostic score can be assigned to the candidate condition of "worsening HF" if supine or prone posture is detected together with an indication of increased respiration exertion, such as an substantial increase in RR or a substantial decrease in TV from their respective reference level. Alternatively, a higher diagnostic score can be assigned to the candidate condition of "worsening HF" if a non-supine posture is detected (such as the tilt angle of posture exceeding a threshold value) during a known or detected sleep state, indicating patient sitting up during sleep to avoid exerted breathing while recumbent.

Rules 604 and 605 can be used for differential diagnosis between worsening of HF and pulmonary disease (such as chronic obstructive pulmonary disease (COPD), pneumonia, or bronchitis. Signal metrics included for the differential diagnosis can include a change in ITTI from a reference value (ΔITTI = ITTI - ITTI_{Ref}), a change in respiration rate (RR) from a reference respiration rate (ΔRR = RR - RR_{Ref}), a rate of change in RR (ΔRR/Δt), and a change in a heart sound (HS) component such as S3 heart sound intensity from a reference level (Δ||S3|| = ||S3|| - ||S3||_{Ref}). According to rule 604, a higher diagnostic score can be assigned to "worsening HF" if (1) ITTI substantially decreases from the reference level by at least a threshold value, which indicates substantial intrathoracic fluid accumulation; (2) RR substantially increases from RR_{Ref} by at least a threshold value, and ΔRR/Δt is within a threshold range, which indicates a gradual onset of increase in respiration rate; and (3) ||S3|| substantially increases from the reference level by at least a threshold value. According to rule 605, a higher diagnostic score can be assigned to "pulmonary disease" if (1) RR substantially increases from the RR_{Ref} by at least a threshold value and ΔRR/Δt exceeds a threshold range, which indicates a sudden onset in rise of respiration rate; or (2) ||S3|| is within a threshold range around ||S3||_{Ref}.

Rule 606 can be used for differentiating diseases like worsening of HF from an infection which may lead to similar pathophysiological manifestations in a patient. The infection can be associated with a portion of the implantable medical device or an implantable lead. The rule 606 can employ signal metrics including a Δ||S3||, ΔITTI, and rate of change in HR, RR, or TV (ΔHR/Δt, ΔRR/Δt, or ΔTV/Δt, respectively). A higher diagnostic score can be assigned to the candidate condition of "device/lead infection" if (1) ΔHR/Δt or ΔRR/Δt is positive and exceeds a threshold, or ΔTV/Δt is negative and exceeds a threshold, indicating respectively a sudden increase in HR or in RR, or sudden decrease in TV; (2) ITTI is within a threshold range around ITTI_{Ref}, indicating non-significant fluid accumulation; and (3) ||S3|| is within a threshold range around ||S3||_{Ref}.

Rule 607 can be used for detecting diastolic HF in a patient. A diastolic heart failure is a condition where a ventricular chamber is unable to accept an adequate volume of blood during diastole at normal diastolic pressures and at volumes sufficient to maintain an appropriate stroke volume. Compared to systolic HF which is usually accompanied with a reduced left ventricular ejection fraction (LVEF), a diastolic HF usually is characterized by a preserved LVEF. The rule 607 can employ a number of signal metrics including, for example, ||S3||, ITTI, TV, or RR, and examine the pattern of onset of changes in these signal metrics. As illustrated at 609, a higher diagnostic score can be assigned to the candidate condition of "worsening of diastolic HF" if (1) Δ||S3||/Δt is positive and exceeds a threshold, indicating a sudden increase of ||S3||, and (2) ΔITTI/Δt or ΔTV/Δt is negative and exceeds a respective threshold, , indicating a sudden decrease of ITTI or sudden decrease of TV.

Rule 608 can be used for differentiating disease such as worsening of HF from sleep disordered breathing (SDB). The rule 608 can include a first timing of a change in apnea-hypopnea index (T_AHI), and a second timing of a change in intrathoracic total impedance value (T_ΔITTI), and a third timing of a change in a HS component such as S3 heart sound intensity (T_Δ||S3||). A higher diagnostic score can be assigned to the candidate condition of "SDB" if T_AHI precedes T_ΔITTI and T_Δ||S3||, which indicates disordered breathing patterns during sleep develop before any significant change in sensors responses such as thoracic impedance or HS intensity.

Rule 609 can be used for used for differential diagnosis between worsening of HF and a ventricular arrhythmia event such as a ventricular tachycardia (VT) or a ventricular fibrillation (VF) episode. The ventricular arrhythmia episode can be detected by an ambulatory medical device. The rule 609 can employ signal metrics including a first timing of the VT or VF episode (T_VT/VF), and a second timing of a change in ITTI (T_ΔITTI) and a third timing of a change in S3 heart sound intensity (T_Δ||S3||). A higher diagnostic score can be assigned to the candidate condition of "VT/VF" if T_VT/VF precedes T_ΔITTI and T_Δ||S3||, which indicates a VT/VF episode is developed before any significant change in sensors responses such as thoracic impedance or HS intensity.

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

Method examples described herein can be machine or computer-implemented at least in part. Some examples can include a computer-readable medium or machine-readable medium encoded with instructions operable to configure an electronic device to perform methods as described in the above examples. An implementation of such methods can include code, such as microcode, assembly language code, a higher-level language code, or the like. Such code can include computer readable instructions for performing various methods. The code may form portions of computer program products. Further, in an example, the code can be tangibly stored on one or more volatile, non-transitory, or non-volatile tangible computer-readable media, such as during execution or at other times. Examples of these tangible computer-readable media can include, but are not limited to, hard disks, removable magnetic disks, removable optical disks (e.g., compact disks and digital video disks), magnetic cassettes, memory cards or sticks, random access memories (RAMs), read only memories (ROMs), and the like.

## Claims

1. A system (200), comprising:
a patient information receiver circuit (201) configured to receive patient information including one or more physiologic signals obtained from a patient;
a physiologic data analyzer circuit (210) configured to detect, from each of the one or more received physiologic signals, a respective physiologic feature indicative of patient physical or physiologic status; and
a differential diagnosis circuit (220) configured to provide a differential diagnosis between worsening heart failure (HF) and one or more candidate conditions, the differential diagnosis circuit configured to:
generate one or more signal metrics using the one or more physiologic features, the one or more signal metrics indicative or correlative of a change in the patient physical or physiologic status;
receive one or more candidate conditions associated with the change in patient physical or physiologic status; and
determine, for worsening HF and the one or more candidate conditions, a respective diagnostic score using the one or more signal metrics, the diagnostic score indicative of the likelihood of the change in the patient's physical or physiologic status being caused by worsening HF or the corresponding one or more candidate conditions.

2. The system of claim 1, wherein the one or more candidate conditions includes a pulmonary disease, a renal disease, an indication of sleep disordered breathing, an atrial or ventricular arrhythmia, an indication of a surgical procedure, or an indication of infection.

3. The system of any one of claims 1 or 2, wherein the differential diagnosis circuit is configured to determine a change or a rate of change of the one or more signal metrics from respective reference level, and to determine the respective diagnostic score in response to the change or the rate of change of the one or more signal metrics respectively meeting a specified criterion.

4. The system of any one of claims 1 through 3, wherein the differential diagnosis circuit is configured to determine the respective diagnostic score using a rule-based model (411).

5. The system of claim 4, wherein the rule-based model includes a multi-level decision tree model, and the differential diagnosis circuit determines the respective diagnostic score in response to the one or more signal metrics respectively meeting a specified criterion.

6. The system of any one of claims 1 through 3, wherein the differential diagnosis circuit determines the respective diagnostic score using at least a temporal relationship between a first signal metric and a different second signal metric.

7. The system of claim 6, wherein the temporal relationship between the first and second signal metrics includes a comparison between a first timing associated with the first signal metric and a second timing associated with the second signal metric.

8. The system of any one of claims 1 through 3 and 6 through 7, wherein the differential diagnosis circuit is configured to determine the respective diagnostic score using a probabilistic model, and wherein the diagnostic score includes a probability value of the patient experiencing, or developing in future, one of the two or more candidate conditions.

9. The system of any one of claims 1 through 8, wherein the differential diagnosis circuit is configured to compute the one or more signal metrics using a plurality of measurements of the physiologic signal feature, the one or more signal metrics including a statistical metric or a morphological metric.

10. The system of any one of claims 1 through 9 wherein the patient information receiver circuit is configured to receive a thoracic impedance signal using an impedance sensor, and the differential diagnosis circuit is configured to compute a change in one or more of a respiration strength measurement, a respiration rate, or a respiration pattern using the thoracic impedance signal.

11. The system of any one of claims 1 through 10, wherein the patient information receiver circuit is configured to receive a heart sound (HS) signal using a heart sound sensor.

12. The system of claim 11, wherein the differential diagnosis circuit is configured to generate one or more signal metrics including intensity of a HS component, the HS component including one or more of an S1, and S2, and S3 or an S4 heart sound.

13. The system of any one of claims 1 through 12, wherein the patient information receiver circuit is configured to receive the patient information including one or more of chronic disease information, prior medical procedure or therapy information, patient demographic information, patient vital sign information, patient symptom information, or patient comorbidity condition.

14. The system of any one of claims 1 through 13, further comprising a user interface configured to provide a presentation of one or more of the detected physiologic features or the diagnostic scores associated with the two or more candidate conditions.

15. The system of any one of claims 1 through 14, wherein:
the differential diagnosis circuit is further configured to determine a most probable diagnosis using a comparison among the diagnostic scores, the most probable diagnosis associated with the highest diagnostic score; and
the user interface is configured to provide the presentation including the most probable diagnosis.

## Patentansprüche

1. System (200), umfassend:
eine Schaltung zum Empfangen von Patienteninformationen (201), die dafür ausgelegt ist, Patienteninformationen zu empfangen, die ein oder mehrere von einem Patienten erhaltene physiologische Signale einschließen;
eine Schaltung zum Analysieren physiologischer Daten (210), die dafür ausgelegt ist, aus dem einen oder aus jedem von den mehreren empfangenen physiologischen Signalen ein jeweiliges physiologisches Merkmal zu erfassen, das einen physischen oder physiologischen Zustand des Patienten angibt; und
eine Differentialdiagnoseschaltung (220), die dafür ausgelegt ist, eine Differentialdiagnose zwischen einer sich verschlimmernden Herzinsuffizienz (HF) und einer oder mehreren in Frage kommenden Erkrankungen bereitzustellen, wobei die Differentialdiagnoseschaltung ausgelegt ist zum:
Erzeugen einer oder mehrerer Signalmetriken unter Verwendung des einen oder der mehreren physiologischen Merkmale, wobei die eine oder die mehreren Signalmetriken auf eine Änderung des physischen oder physiologischen Zustands des Patienten hinweisen oder damit korreliert sind;
Empfangen einer oder mehrerer in Frage kommender Erkrankungen, die mit der Änderung des physischen oder physiologischen Zustands des Patienten assoziiert sind; und
Bestimmen eines jeweiligen diagnostischen Scores für eine sich verschlimmernde HF und für die eine oder die mehreren in Frage kommenden Erkrankungen unter Verwendung der einen oder der mehreren Signalmetriken, wobei der diagnostische Score die Wahrscheinlichkeit dafür angibt, dass die Änderung des physischen oder physiologischen Zustands des Patienten durch eine sich verschlimmernde HF oder die entsprechende eine oder mehrere in Frage kommende Erkrankungen verursacht wird.

2. System nach Anspruch 1, wobei die eine oder die mehreren in Frage kommenden Erkrankungen eine Lungenkrankheit, eine Nierenkrankheit, einen Hinweis auf eine schlafbezogene Atmungsstörung, eine atriale oder ventrikuläre Arrhythmie, einen Hinweis auf einen chirurgischen Eingriff oder einen Hinweis auf eine Infektion einschließen.

3. System nach einem der Ansprüche 1 oder 2, wobei die Differentialdiagnoseschaltung dafür ausgelegt ist, eine Änderung oder eine Änderungsrate der einen oder der mehreren Signalmetriken gegenüber einem jeweiligen Bezugsniveau zu bestimmen und als Reaktion darauf, dass die Änderung oder die Änderungsrate der einen oder der mehreren Signalmetriken ein bestimmtes Kriterium erfüllt, den jeweiligen diagnostischen Score zu bestimmen.

4. System nach einem der Ansprüche 1 bis 3, wobei die Differentialdiagnoseschaltung dafür ausgelegt ist, den jeweiligen diagnostischen Score unter Verwendung eines regelbasierten Modells (411) zu bestimmen.

5. System nach Anspruch 4, wobei das regelbasierte Modell ein mehrschichtiges Entscheidungsbaummodell einschließt und die Differentialdiagnoseschaltung den jeweiligen diagnostischen Score als Reaktion darauf bestimmt, dass die eine oder die mehreren Signalmetriken jeweils ein bestimmtes Kriterium erfüllen.

6. System nach einem der Ansprüche 1 bis 3, wobei die Differentialdiagnoseschaltung den jeweiligen diagnostischen Score unter Verwendung zumindest einer zeitlichen Beziehung zwischen einer ersten Signalmetrik und einer anderen, zweiten Signalmetrik bestimmt.

7. System nach Anspruch 6, wobei die zeitliche Beziehung zwischen der ersten und der zweiten Signalmetrik einen Vergleich zwischen einem ersten Zeitwert, der mit der ersten Signalmetrik assoziiert ist, und einem zweiten Zeitwert, der mit der zweiten Signalmetrik assoziiert ist, einschließt.

8. System nach einem der Ansprüche 1 bis 3 und 6 bis 7, wobei die Differentialdiagnoseschaltung dafür ausgelegt ist, den jeweiligen diagnostischen Score unter Verwendung eines probabilistischen Modells zu bestimmen, und wobei der diagnostische Score einen Wahrscheinlichkeitswert dafür einschließt, dass der Patient eine von den zwei oder mehr in Frage kommenden Erkrankungen aufweist oder künftig entwickeln wird.

9. System nach einem der Ansprüche 1 bis 8, wobei die Differentialdiagnoseschaltung dafür ausgelegt ist, die eine oder die mehreren Signalmetriken unter Verwendung einer Vielzahl von Messungen des Merkmals des physiologischen Signals zu berechnen, wobei die eine oder die mehreren Signalmetriken eine statistische Metrik oder eine morphologische Metrik einschließen.

10. System nach einem der Ansprüche 1 bis 9, wobei die Schaltung zum Empfangen von Patienteninformationen dafür ausgelegt ist, ein thorakales Impedanzsignal unter Verwendung eines Impedanzsensors zu empfangen, und die Differentialdiagnoseschaltung dafür ausgelegt ist, eine Änderung an einem oder mehreren von einer Respirationsstärkemessung, einer Respirationsrate oder einem Respirationsmuster unter Verwendung des thorakalen Impedanzsignals zu berechnen.

11. System nach einem der Ansprüche 1 bis 10, wobei die Schaltung zum Empfangen von Patienteninformationen dafür ausgelegt ist, ein Herzton(HT)-Signal unter Verwendung eines Herzsensors zu empfangen.

12. System nach Anspruch 11, wobei die Differentialdiagnoseschaltung dafür ausgelegt ist, eine oder mehrere Signalmetriken, einschließlich einer Intensität einer HT-Komponente, zu erzeugen, wobei die HT-Komponente eines oder mehrere von einem S1- und S2- und S3- oder einem S4-Herzton einschließt.

13. System nach einem der Ansprüche 1 bis 12, wobei die Schaltung zum Empfangen von Patienteninformationen dafür ausgelegt ist, die Patienteninformationen zu empfangen, die eines oder mehrere von Informationen zu einer chronischen Krankheit, Informationen zu einem medizinischen Verfahren oder einer Therapie, demographische Informationen über den Patienten, Informationen über Vitalzeichen des Patienten, Informationen über Symptome des Patienten oder einen Comorbiditätsstatus des Patienten einschließen.

14. System nach einem der Ansprüche 1 bis 13, ferner eine Benutzeroberfläche umfassend, die dafür ausgelegt ist, eine Präsentation eines oder mehrerer von den erfassten physiologischen Merkmalen oder den diagnostischen Scores, die mit den zwei oder mehr in Frage kommenden Erkrankungen assoziiert sind, bereitzustellen.

15. System nach einem der Ansprüche 1 oder 14, wobei:
die Differentialdiagnoseschaltung ferner dafür ausgelegt ist, eine wahrscheinlichste Diagnose unter Verwendung eines Vergleichs zwischen den diagnostischen Scores zu bestimmen, wobei die wahrscheinlichste Diagnose mit dem höchsten diagnostischen Score assoziiert ist; und
die Benutzeroberfläche dafür ausgelegt ist, die Präsentation einschließlich der wahrscheinlichsten Diagnose bereitzustellen.

## Revendications

1. Système (200), comprenant :
un circuit de récepteur d'information de patient (201) qui est configuré pour recevoir une information de patient qui inclut un signal ou plusieurs signaux physiologique(s) qui est/sont obtenu(s) à partir d'un patient ;
un circuit d'analyseur de données physiologiques (210) qui est configuré pour détecter, à partir de chacun des un ou plusieurs signaux physiologiques reçus, une caractéristique physiologique respective qui est indicative d'un état physique ou physiologique du patient ; et
un circuit de diagnostic différentiel (220) qui est configuré pour fournir un diagnostic différentiel entre une aggravation de défaillance cardiaque (HF) et une ou plusieurs maladie(s) candidate(s), le circuit de diagnostic différentiel étant configuré pour :
générer un ou plusieurs paramètre(s) métrique(s) de signal en utilisant les une ou plusieurs caractéristiques physiologiques, les un ou plusieurs paramètre(s) métrique(s) de signal étant indicatifs d'une modification de l'état physique ou physiologique du patient ou étant en corrélation avec cette même modification ;
recevoir une ou plusieurs maladie(s) candidate(s) qui est/sont associée(s) à la modification de l'état physique ou physiologique du patient ; et
déterminer, pour une aggravation de HF et pour les une ou plusieurs maladies candidates, un score de diagnostic respectif en utilisant les un ou plusieurs paramètre(s) métrique(s) de signal, le score de diagnostic étant indicatif de la vraisemblance du fait que la modification de l'état physique ou physiologique du patient soit générée par l'aggravation de HF ou par les une ou plusieurs maladies candidates correspondantes.

2. Système selon la revendication 1, dans lequel les une ou plusieurs maladies candidates incluent une pneumopathie, une néphropathie, une indication de troubles respiratoires du sommeil, une arythmie auriculaire ou ventriculaire, une indication d'une procédure chirurgicale ou une indication d'infection.

3. Système selon l'une quelconque des revendications 1 ou 2, dans lequel le circuit de diagnostic différentiel est configuré pour déterminer une variation ou un taux de variation des un ou plusieurs paramètre(s) métrique(s) de signal par rapport à un niveau de référence respectif, et pour déterminer le score de diagnostic respectif en réponse au fait que la variation ou le taux de variation des un ou plusieurs paramètre(s) métrique(s) de signal satisfait respectivement un critère spécifié.

4. Système selon l'une quelconque des revendications 1 à 3, dans lequel le circuit de diagnostic différentiel est configuré pour déterminer le score de diagnostic respectif en utilisant un modèle basé sur règle(s) (411).

5. Système selon la revendication 4, dans lequel le modèle basé sur règle(s) inclut un modèle par arborescence décisionnelle à multiples niveaux, et le circuit de diagnostic différentiel détermine le score de diagnostic respectif en réponse au fait que les un ou plusieurs paramètre(s) métrique(s) de signal satisfont respectivement un critère spécifié.

6. Système selon l'une quelconque des revendications 1 à 3, dans lequel le circuit de diagnostic différentiel détermine le score de diagnostic respectif en utilisant au moins une relation temporelle entre un premier paramètre métrique de signal et un second paramètre métrique de signal différent.

7. Système selon la revendication 6, dans lequel la relation temporelle entre les premier et second paramètres métriques de signal inclut une comparaison entre une première temporisation qui est associée au premier paramètre métrique de signal et une seconde temporisation qui est associée au second paramètre métrique de signal.

8. Système selon l'une quelconque des revendications 1 à 3 et 6 et 7, dans lequel le circuit de diagnostic différentiel est configuré pour déterminer le score de diagnostic respectif en utilisant un modèle probabiliste, et dans lequel le score de diagnostic inclut une valeur de probabilité que le patient éprouve, ou développe dans le futur, l'une des deux maladies candidates ou plus.

9. Système selon l'une quelconque des revendications 1 à 8, dans lequel le circuit de diagnostic différentiel est configuré pour calculer les un ou plusieurs paramètre(s) métrique(s) de signal en utilisant une pluralité de mesures de la caractéristique de signal physiologique, les un ou plusieurs paramètre(s) métrique(s) de signal incluant un paramètre métrique statistique ou un paramètre métrique morphologique.

10. Système selon l'une quelconque des revendications 1 à 9, dans lequel le circuit de récepteur d'information de patient est configuré pour recevoir un signal d'impédance thoracique en utilisant un capteur d'impédance, et le circuit de diagnostic différentiel est configuré pour calculer une variation d'un ou de plusieurs paramètre(s) pris parmi une mesure de puissance de respiration, un rythme de respiration et un profil de respiration en utilisant le signal d'impédance thoracique.

11. Système selon l'une quelconque des revendications 1 à 10, dans lequel le circuit de récepteur d'information de patient est configuré pour recevoir un signal de bruit du cœur (HS) en utilisant un capteur de bruit du cœur.

12. Système selon la revendication 11, dans lequel le circuit de diagnostic différentiel est configuré pour générer un ou plusieurs paramètre(s) métrique(s) de signal incluant l'intensité d'une composante de HS, la composante de HS incluant un ou plusieurs bruit(s) du cœur pris parmi les bruits du cœur S1, S2, S3 et S4.

13. Système selon l'une quelconque des revendications 1 à 12, dans lequel le circuit de récepteur d'information de patient est configuré pour recevoir l'information de patient qui inclut une ou plusieurs information(s) prise(s) parmi une information de maladie chronique, une information de procédure ou de thérapie médicale antérieure, une information démographique de patient, une information de signe vital de patient, une information de symptôme(s) de patient et une condition de comorbidité de patient.

14. Système selon l'une quelconque des revendications 1 à 13, comprenant en outre une interface utilisateur qui est configurée pour fournir une présentation d'une ou de plusieurs des caractéristiques physiologiques détectées ou d'un ou de plusieurs des scores de diagnostic qui sont associé(e)s aux deux maladies candidates ou plus.

15. Système selon l'une quelconque des revendications 1 à 14, dans lequel :
le circuit de diagnostic différentiel est en outre configuré pour déterminer un diagnostic le plus probable en utilisant une comparaison entre les scores de diagnostic, le diagnostic le plus probable étant associé au score de diagnostic le plus élevé ; et
l'interface utilisateur est configurée pour fournir la présentation qui inclut le diagnostic le plus probable.
